# EUROPEAN PATENT SPECIFICATION

(11) **EP 4 168 111 B1**
(45) Date of publication and mention of the grant of the patent: **05.11.2025**
(21) Application number: 21826212.9
(22) Date of filing: 16.06.2021
(51) Int. Cl.: A61N 5/06, G16H 20/40, G16H 40/40, A61B 1/04

(54) **SYSTEM FOR THERAPEUTIC GASTROINTESTINAL PHOTOBIOMODULATION**
SYSTEM ZUR THERAPEUTISCHEN GASTROINTESTINALEN PHOTOBIOMODULATION
SYSTÈME DE PHOTOBIOMODULATION GASTRO-INTESTINALE THÉRAPEUTIQUE

(30) Priority: 17.06.2020 US 202063039985 P
(43) Date of publication of application: 26.04.2023
(73) Proprietor: Niche Biomedical, Inc., Los Angeles, CA 90024 (US)
(72) Inventor: BALDWIN, Alexander Barnes, Santa Monica, California 90405 (US); LIU, Chien-Pan, Tainan City, 701039 (TW); WANG, Po-Min, Fremont, California 94538 (US); LO, Yi-Kai, Culver City, California 90230 (US)
(74) Representative: Marks & Clerk LLP
(86) International application number: PCT/US2021/037559
(87) International publication number: WO 2021/257656

(56) References cited:
- US-A1- 2006 195 014
- US-A1- 2008 058 587
- US-A1- 2009 048 583
- US-A1- 2009 048 583
- US-A1- 2013 053 657
- US-A1- 2013 053 928
- US-A1- 2013 053 928
- US-A1- 2014 005 758
- US-A1- 2014 005 758

## Description

### TECHNICAL FIELD

This invention relates generally to intraluminal photobiomodulation ("PBM") and more specifically to a system that can be used for therapeutic gastrointestinal PBM

### BACKGROUND

The term photobiomodulation ("PBM") refers to the use of electromagnetic radiation of light (traditionally with one or more wavelengths in the ultraviolet ("UV"), visible, or infrared ("IR") portions of the spectrum) to modulate a biological process and produce one or more therapeutic effects. PBM is advantageous over traditional medical or surgical therapies because PBM can produce the one or more therapeutic effects while being safe, without producing a systemic effect or any associated side effects. Traditionally, PBM has been applied through the skin. However, traditional PBM has proven ineffective in providing therapeutic effects for many conditions.

Application of PBM through the skin has proven ineffective to treat internal areas of the body because the light used for PBM has a limited penetration depth (often on the order of centimeters). The gastrointestinal ("GI") tract, in particular, is situated under several muscle and tissue layers of the abdominal wall and beyond the penetration depth of the light of traditional PBM. To treat areas of the GI tract not reached by traditional PBM effectively, and to decrease the associated cost and burden, a new delivery scheme for PBM is necessary.

US 2013/053928 discloses a wireless in vivo therapeutic devices equipped with one or more LEDs for radiating the pathological areas, comprising a bleeding detection sensing head including a light detector. A processor, external to the device, may process the signal sent by the light detector and create an absorption or transmission spectra of the in vivo fluids.

### SUMMARY

The invention relates to a system as defined in the appended claims. Embodiments, examples or aspects in the following disclosure, in particular methods, which do not fall under the scope of the claims are presented for illustration purposes only and do not form part of the invention.

A method and system for therapeutic gastrointestinal PBM are described, decreasing the associated cost and burden, while increasing the penetration depth of the light. The method and system would be highly beneficial to patients suffering from peptic ulcers, bowel resection, or inflammation of the GI tract, as well as patients suffering from other diseases of the GI tract.

The method includes delivering photobiomodulation therapy to a portion of a patient's GI tract by at least one light delivery device; determining, by a signal processing device, whether the photobiomodulation therapy is sufficient to treat the portion of the patient's GI tract based on a reflectance signal detected by at least one photodetector; and at least one of: reconfiguring the photobiomodulation therapy; or expelling the at least one light delivery device, the at least one photodetector, and the signal processor from the GI tract by defecation.

The system includes an intraluminal device and an external device. The intraluminal device includes at least one light delivery device configured to provide photobiomodulation; at least one photodetector device configured to measure reflectance based on the photobiomodulation; a processor configured to receive a reflectance signal from the at least one photodetector device based on the reflectance measured by the at least one photodetector device and process the reflectance signal; and a wireless transceiver coupled to the processor to transmit at least a portion of the processed reflectance signal. The external device includes at least a wireless transceiver configured to receive the at least the portion of the processed reflectance signal.

### BRIEF DESCRIPTION OF THE DRAWINGS

The foregoing and other features of the present disclosure will become apparent to those skilled in the art to which the present disclosure relates upon reading the following description with reference to the accompanying drawings, in which:
FIG. 1 is an illustration of a system that can be used for therapeutic gastrointestinal ("GI") photobiomodulation ("PBM");
FIG. 2 is an illustration of an example of a PBM pill, which can be used in the system of FIG. 1
FIG. 3 is a block diagram of the system of FIG. 1;
FIG. 4 is graphical representation of the capability of the system of FIG. 1 to provide light stimulation at a plurality of wavelengths, intensities, and durations, independently and simultaneously;
FIG. 5 is an illustration of another example of a PBM pill, which can be used in the system of FIG. 1;
FIG. 6 is a schematic of an LED driver system with an ASIC that can be used by the PBM pill of FIG. 5;
FIG. 7 is a schematic of an LED current controller that can be used by the PBM pill of FIG. 5;
FIG. 8 is a schematic of a circuit, with may be connected to the photodetector of the PBM pill of FIG. 5, designed to filter out both the AC and DC signals and extract them for ADC analysis;
FIG. 9 is a block diagram schematic of hardware that may be used by the PBM pill of FIGS. 3 or 5;
FIG. 10 is a block diagram schematic of the circuit powering at least a portion of the PBM pill of FIGS. 3 or 5;
FIG. 11 is an illustration of a pill case that can be used with the PBM pill of FIGS. 3 or 5 to attach the PBM pill to a wall of the GI tract;
FIG. 12 is an illustration of an intraluminal device package that can be used with the PBM pill of FIGS. 3 or 5;
FIG. 13 is an illustration of an example of the system of FIG. 1 with multiple PBM pills of FIGS. 3 or 5 being used at different locations in a patient's GI tract;
FIG. 14 is an illustration of the internal components of the PBM pill of FIG. 1;
FIG. 15 is a graphical representation of test pulses between therapeutic pulses used to determine a level of tissue oxygenation;
FIG. 16 is a block diagram showing an example of how a dirty photodetector signal may be cleaned;
FIG. 17 is an example of the system of FIG. 1 in use, where a device that applies a light signal is placed on a patient's skin above the location of treatment to provide therapy;
FIG. 18 is a flow diagram of a method for accelerating recovery after bowel resection surgery;
FIG. 19 is a flow diagram of a method for treating inflammatory bowel disease;
FIG. 20 is a flow diagram of a method for treating gastrointestinal ulcers; and
FIG. 21 is a flow diagram of a method for treating bacterial overgrowth.

### DETAILED DESCRIPTION

This disclosure relates generally to a new delivery scheme to deliver photobiomodulation ("PBM") to the gastrointestinal ("GI") tract for therapeutic purposes. This new delivery scheme enables selective biomodulation of GI tissues by applying PBM using light at appropriate power levels and wavelengths to provide therapy for a variety of disorders, conditions, and other medical issues (both major and minor) affecting one or more components of the GI tract. The new delivery scheme is advantageous over previous and hypothetical solutions for delivering PBM to the GI tract that include some variation of inserting a fiber optic endoscopic device attached to a light source outside of the body into the GI tract. These solutions require expensive and invasive procedures, leading to a prohibitive cost and undue burden on patients. Advantageously, the delivery scheme described herein minimizes the cost and burden on the patients while enabling selective biomodulation of GI tissues using PBM.

The GI tract, which is employed by the body to process food and absorb nutrients, includes components like the esophagus, the stomach, the small intestine, the large intestine (colon), and the rectum. The esophagus connects the mouth and stomach and produces peristaltic contractions that push food distally. Esophageal mucosa includes of stratified squamous epithelium organized into three layers of squamous cells; surrounding the epithelium are muscle fibers to enable peristaltic contraction. The proximal third of the esophagus contains striated muscle, while the distal two thirds of the esophagus includes smooth muscle. The esophagus is innervated by the vagus nerve and the cervical and thoracic sympathetic trunk; of these, the vagus nerve plays the primary role in mediating the initiation of peristalsis.

The stomach is located below the diaphragm at the other side of the lower esophageal sphincter from the esophagus and serves to aid in digestion through the acid- and enzyme-mediated breakdown of chemical compounds in food. The stomach is distensible and can hold one liter of food in an average adult. The stomach is lined with gastric glands which secrete hydrochloric acid and the digestive enzyme pepsin. Hydrochloric acid production lowers the pH to around 2, which is necessary to prevent bacterial infection and for protease activation. Muscles surrounding the stomach include the inner oblique layer, which creates a churning motion that physically breaks down food; the middle circular layer, which controls movement of digested food into the duodenum; and the outer longitudinal layer, which produces peristaltic contractions to move food towards the end of the stomach. Muscle activation and movement is controlled by the autonomic nervous system. The distal end of the stomach contains the pyloric sphincter, which controls the passage of digested food into the small intestine.

The small intestine absorbs nutrients and minerals from the products of stomach digestion. The small intestine includes of the duodenum, jejunum, and ileum; generally, the duodenum neutralizes the acidic product of stomach digestion and combines it with digestive enzymes from the pancreas and liver, while the jejunum and ileum contain villi which are optimized for the absorption of various nutrients. The walls of the small intestine includes, from the inner lumen out, the mucosa, submucosa, muscular layer, and serosa. The mucosa includes simple columnar epithelium optimized for nutrient absorption. Submucosa includes a dense and irregular layer of blood vessels, lymphatic tissue, and nerves; the muscular layer is arranged in a circular pattern around the small intestine and provides peristaltic contractions to move digestive products through the GI tract; the serosa provides connectivity to the rest of the body and prevents friction between adjacent intestinal sections. The small intestine is innervated by the enteric nervous system, which can act independent of the central nervous system but is modulated by the vagus nerve. The small intestine transitions to the colon at the ileocecal junction. The colon absorbs any remaining water and salts before process the remaining waste products of digestion into feces. The colon also serves as a home for beneficial bacterial colonies which ferment unabsorbed food material. The colonic wall is lined with simple columnar epithelium pocketed with pits known as colonic crypts; these crypts contain stem cells which continuously produce the cells lining the colon. After passing through the colon, fecal materials travels to the rectum and is expelled from the body.

As noted, PBM can be used to treat conditions (also referred to as major medical issues) of one or more components of the GI tract. Several major medical issues involve disorders of the GI tract including, but not limited to gastrointestinal ulcers, inflammatory bowel diseases, and small intestinal bacterial overgrowth. In addition, a high number of patients must undergo bowel resection surgery, the recovery from which presents risk of complications and reduced quality of life. Unfortunately, many complications exist during and after bowel resection surgery. These complications may include post-operative ileus (POI), anastomotic leakage (AL), and hemorrhage.

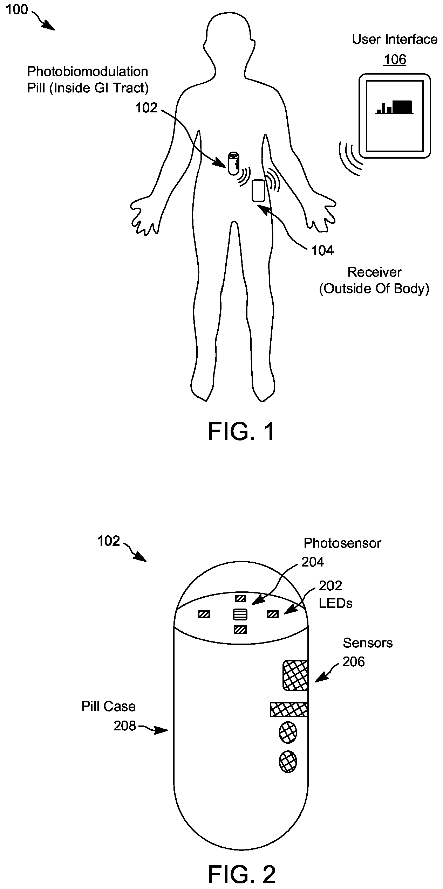

FIG. 1 provides an illustration of a system 100 that can be used to modulate various biological parameters within the GI tract using the applied for therapy. Key features of the system 100 include (1) the ability to apply therapeutic PBM to biological tissue, including but not limited to the interior lumen of the GI tract, (2) the ability to measure reflectance during PBM to enable closed-loop therapy, and (3) the intraluminal form factor's ability to allow therapeutic intervention outside the clinic and without any surgical intervention. Many aspects of the system 100 can be particularly advantageous for various clinical applications, such as the non-limiting examples of: (1) the modulation of GI tissue after bowel resection surgery to promote revascularization and/or accelerate wound healing, (2) the selective modulation of inflamed GI tissue to reduce inflammation in patients with an inflammatory bowel disease, (3) the detection and therapeutic modulation of ulcerated GI tissue, and 4) the detection and treatment of bacterial overgrowth in the GI tract, particularly the intestines. Furthermore, the system 100 can be used for diagnostic purposes such as: (1) monitoring GI tissue perfusion, and (2) remotely monitoring parameters, such as heart rate and respiratory rate.

The system 100 includes one or more components that are inside the GI tract (e.g., PBM pill 102) and one or more components that are outside the body (e.g., receiver 104 and user interface 106). The PBM pill 102 can be configured to be positioned within the GI tract as an intraluminal device and can include a biocompatible package enclosing at least (1) one or more light delivery components configured to deliver light to surrounding biological tissue at a range of intensities and/or at one or more of a variety of wavelengths, (2) one or more light detection components configured to measure reflected light off the biological tissue at one or more wavelengths, and (3) a processing device capable of communicating with the one or more light delivery components and the one or more light detection components. The processing device can instruct the one or more light delivery components of parameters for the light delivery (e.g., wavelength, time of application, on time, off time, intensity, or the like), receive reflectance signals, and/or perform signal processing techniques. For example, the signal processing techniques can include executing signal analysis and/or other processing algorithms (which may be stored in a non-transitory memory affiliated with the processing device). The biocompatible package can withstand the corrosive environment inside the GI tract unimpeded and without substantial degradation.

The PBM pill 102 can be wirelessly connected to the receiver 104 and/or the user interface device 106. Accordingly, the components of the PBM pill 102 can further include a wireless transmitter or a wireless transceiver (with transmission and reception capabilities). The receiver 104 and/or the user interface device 106 can be configured to receive reflectance data (based on the reflectance signal) and provide a visualization of the reflectance data. Additionally, the receiver 104 and/or the user interface device 106 can control at least one parameter of the applied light therapy (e.g., the intensity, the wavelength, the time, etc.). The PBM pill 102, the receiver 104, and/or the user interface device 106 can further include transducers for monitoring GI parameters such as temperature, pH, and/or pressure. These measurements may be used for various purposes, including but not limited to (1) calibrating the photosensor and LEDs, (2) monitoring the location of the intraluminal device in order to activate various modalities in various GI tract sections, and (3) monitoring the effects of PBM on the GI environment to prevent unwanted side effects, such as tissue heating.

An example configuration of the PBM pill 102 (also referred to as the GI pill or the intralumenal device) is shown in FIG. 2. The PBM pill 102 can have one or more light sources 202, such as light emitting diodes or laser diodes (collectively referred to as LEDs) to emit the light (e.g., one or more wavelengths of light), one or more photosensors 204 (also referred to as photodetectors, as well as specific circuit parts, such as photodiodes, photoresistors, phototransistors, or the like) to detect reflected light (e.g., by/from/off of tissue), and one or more sensors 206 configured to detect one or more properties of the GI tract, all at least partially within in a pill case 208. The one or more LEDs 202 can be in an array, which can be on the exterior of the PBM pill 102, within a hole in the pill case 208, or within a transparent (also referred to as see-through) portion of the pill case 208. In some instances, the one or more photosensors 204 can be similarly positioned. The pill case 208 can be constructed of a biocompatible material that can help the PBM pill 102 pass through the GI tract without causing obstruction.

The PBM pill 102 can include the pill case 208 encapsulating the LED array 302 (including the one or more light sources 202), one or more photosensors 304 (including the one or more photosensors 204), a signal processor 306 (including signal processing electronics), and a transducer & antenna 308. The signal processor 306 can include signal processing electronics capable of recording reflectance measurements from the photosensor 304 and modulating parameters of the light emitted by the LED array 302 (e.g., intensity, wavelengths, time applied, on time, off time, frequency, etc.). The GI pill also can include a power source (not illustrated) capable of providing power to the LED array 302, the one or more photosensors 304 (please note that elements 202 and 302 are used interchangeably to describe the LEDs and 204 and 304 are used interchangeably to describe the photodetectors), and the signal processor 306 (and may provide power also to the transducer & antenna 308). It should be noted that one or more external components can also have a transducer & antenna 310, which can pair with the internal transducer & antenna 308 of the PBM pill. The external components can also include a computer 312, a user interface 314, as well as a secure cloud 316 in communication with the computer 312.

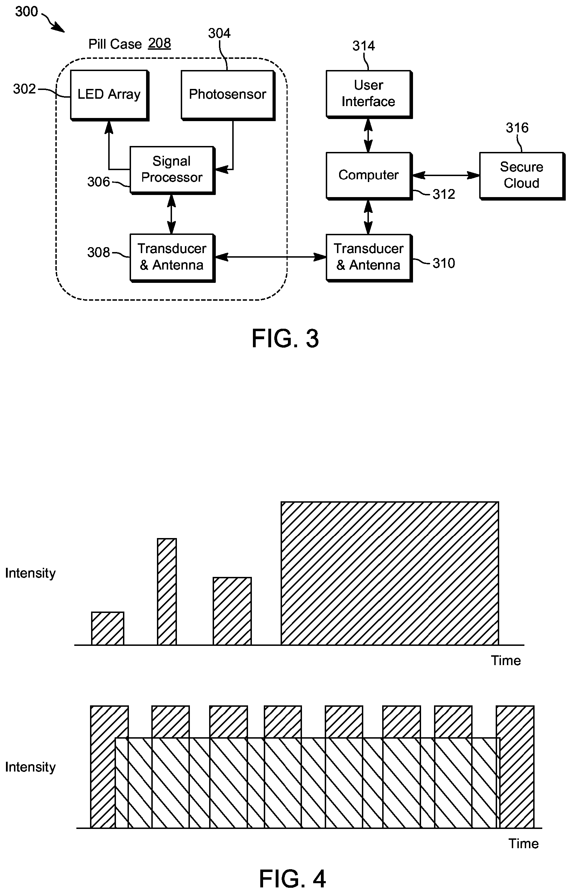

As noted, the LED array 302 can provide the therapeutic light to the interior of the GI tract. The LEDs of the array may be configured to emit light in the ultraviolet (10nm to 400nm wavelength), visible (400nm to 700nm wavelength), and/or infrared (700nm to 1100nm wavelength) ranges. As one example, the LED array 302 can include four LEDs, at least one of which can be configured to emit light at a different wavelength than the other LEDs. As another example, the LED array 302 can include four LEDs, each configured to emit light at a different wavelength (e.g., 405nm, 635nm, 850nm, and 940nm). The LED array 302 can include multiple types of LEDs designed to emit at different wavelengths; in this way, various combinations of LEDs within the array can be activated simultaneously to deliver multispectral wavelength light, light at a single wavelength, or some sequential combination thereof (reflected, for example, in FIG. 4, which shows that the system 100 can provide light stimulation at a plurality of wavelengths, intensities, durations, etc., independently). Each element in the LED array 302 can be independently addressable, allowing the spectral profile and intensity of light emitted by the array to be finely tuned by activating various elements of the LED array 302. The power delivered to the entire LED array 302 can be modulated over a range to control the magnitude of the therapeutic effect. It should be noted that the LED array 302 can have any spatial configuration. Example spatial configurations include being configured such that light is directed proximally up the GI tract, distally down the GI tract, or laterally at the walls of the GI tract.

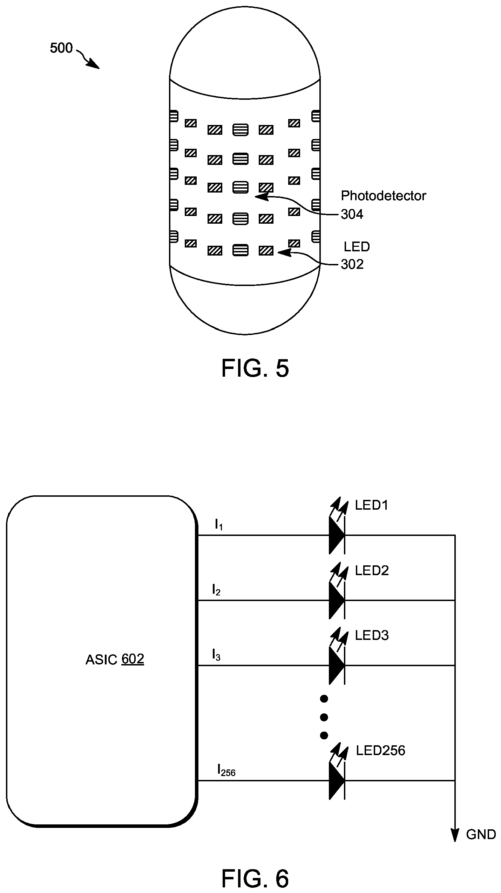

One or more of the photosensors 304 can be configured to measure the magnitude or intensity of the light reflected off of tissue during PBM. As an example, one of the photosensors 304 can be placed in the center of the four LEDs in the array and optically isolated from the surrounding LEDs. The one or more photosensors can be positioned on the proximal end, distal end, or laterally around the PBM pill 102. As shown in FIG. 5, multiple photosensors 304 at various positions (portions of which may be sensitive to a different wavelength of light or different wavelengths of light) can be used to measure reflectivity in multiple directions from the LEDs 302 of the PBM pill. It should be noted that in FIG. 5, the LED array 302 can be placed laterally around the circumference of the PBM pill 102 with one or more photodetectors 304 around the circumference of the PBM pill 102, allowing for targeted PBM of a particular sections of the tissue of the GI tract.

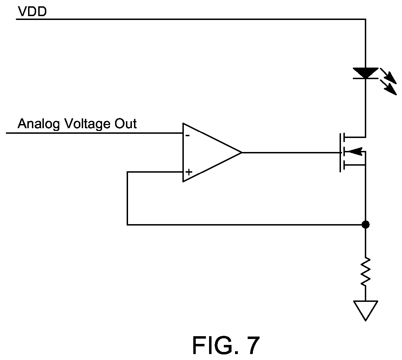

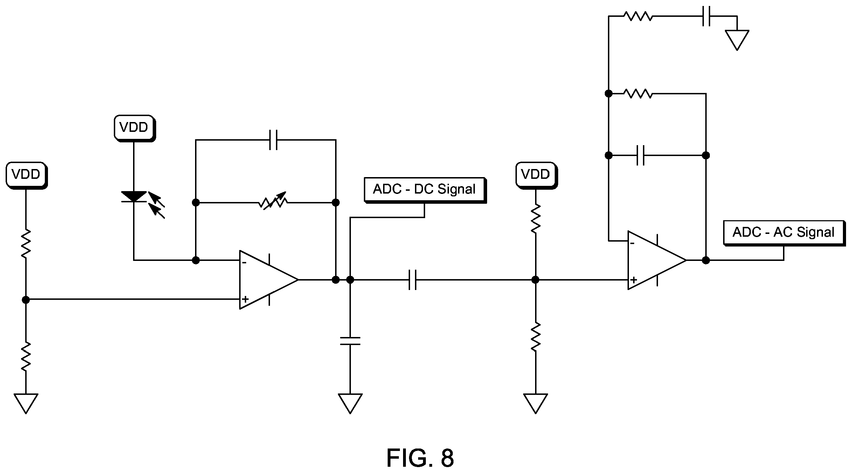

The LED array 302 and photodiodes 304 can be connected to an electrical circuit for control and signal processing (e.g., signal processor 306). The circuit may contain a microcontroller, LED driver circuitry, analog front-end circuitry for the photodiode and/or the transducer & antenna 308. In the example shown in FIG. 6, the LED driver circuitry can include a custom ASIC 602 able to generate precise amounts of current; this ASIC 602 is directly connected to the LEDs and is used to provide independent control of the amplitude, pulse width, and frequency of each LED. In another example (shown in FIG. 7) the current through each LED may be independently controlled by placing an n-MOS transistor in series with the LED and a resistor, with an amplifier configured such that the output of the amplifier was connected to the gate of the transistor, the negative terminal is connected to the source of the transistor, and the positive terminal is connected to an analog voltage source; by varying the analog voltage, a precise current can be generated through the LED. As shown, for example in FIG. 8, the photodiode can be connected to a transimpedance amplifier composed of an amplifier, a capacitor, and a digitally controlled resistor configured to convert the current produced by the photodiode to a voltage. This voltage is then sent to an analog-digital converter. In addition, this signal is sent through a two-stage active bandpass filter configured to isolate the AC component of the signal. Both the initial voltage signal and the AC component of the signal may be digitized simultaneously by analog-digital converters. A digital potentiometer may be adjusted dynamically using a digital PID controller to keep the first signal within a predetermined range while using the AC component of the signal to for diagnostic purposes.

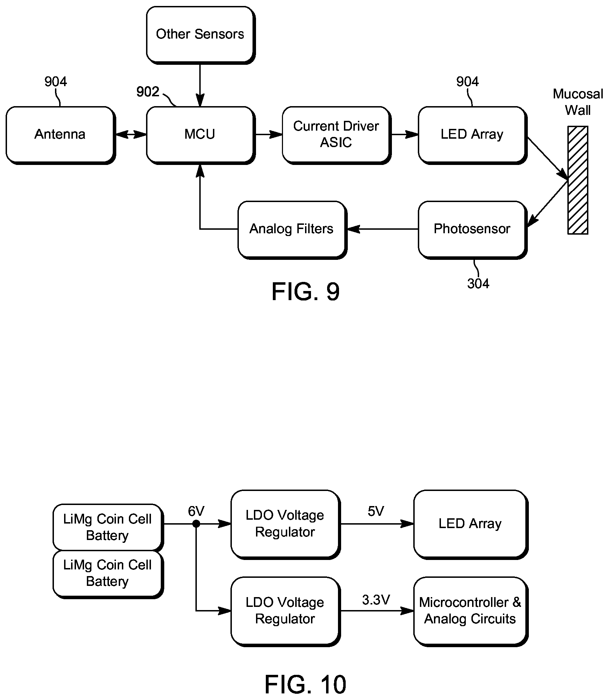

Control of the LED array 302 and processing of the reflectance measurements from the photodetector 304 as shown in FIG. 9 are performed by an embedded microcontroller (e.g., MCU 902), which is coupled to a wireless transceiver able to modulate an antenna 904 (similar to the transducer & antenna 308) inside the PBM pill 102. The antenna 904 and microcontroller 902 may be packaged into a single system on chip, for example, the ISP 1807 miniaturized Bluetooth system. The antenna 904 may be configured to transmit and receive data using one of a number of frequencies and standards, including Bluetooth or Bluetooth Low Energy (BLE); one or multiple MICS bands between 400 MHz and 457 MHz; or WiFi at 2.4 MHz or 5 MHz. The electronics can be programmed to modulate one or more parameters of the LED array 302, including the wavelength, power, and/or timing of the emitted light, in response to measurements from the one or more photodiode 304. The MCU 902 can transmit photodiode 304 measurement outside of the body using the antenna 904 and receive control signals for the LED array 302 using the same antenna 904.

The power source of the PBM pill 102 can be a battery. The battery may be one of various chemistries including but not limited to lithium ion, lithium polymer, lithium magnesium, nickel cadmium, or silver oxide. For example, the battery includes two lithium magnesium coin cells configured to output 6 volts, and connected to two independent voltage regulators to provide a higher voltage for the LED array and a lower voltage to power the microcontroller and analog circuits (e.g., shown in FIG. 10). The battery may be rechargeable by power delivered to the body through the antenna, or it may be disposable. In another example, the power source can include a second antenna configured to receive power from another antenna external to the body to rectify the power to provide power for the circuits. This antenna (the transceiver and antenna of the PBM pill 102) may receive power over a radiofrequency or inductive wireless link. In another example, the power source may include a capacitor which is configured to store energy for use by the LEDs and the circuit.

The LED array 302, one or more photodiodes 304, processor 306, and power source can be placed within a biocompatible package (also referred to as a pill case) designed to pass through the GI tract without causing obstruction. The package may be shaped like a pill. The package can also be any shape with a size and dimensions such that the package can be swallowed and may pass through the gastrointestinal tract while minimizing the chance of obstruction or stiction. For example, the package can have a cylindrical center section with semicircular domes on either end, constructed with length of 27mm and diameter of 12mm (however, the dimensions may change). The package may be manufactured out of one or more biocompatible materials such as titanium, ceramic, plastic, or glass. For example, the package can be made of ABS, which is biocompatible and stable in the acidic environment of the stomach. The package can include one or more optically transparent sections or other types of sections to allow radiation from the LED array 302 to exit the package and reflected light to enter the package and modulate the one or more photodiodes 304. The package may be coated with a transparent material to facilitate biocompatibility, such as Parylene C or silicone.

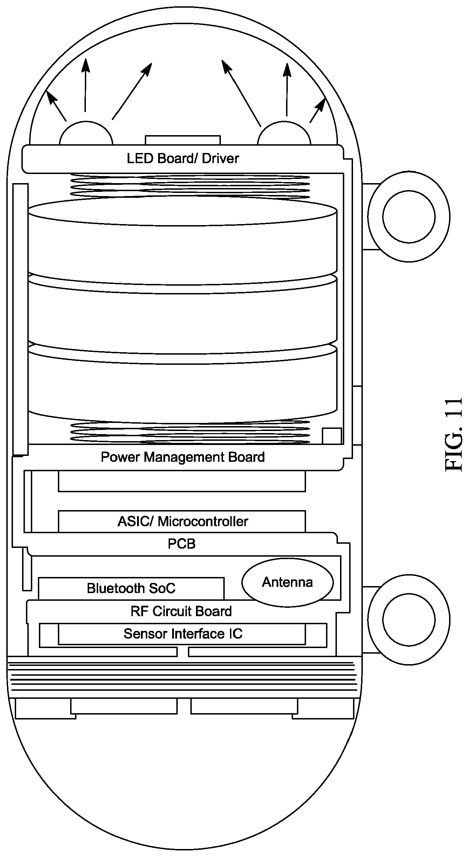

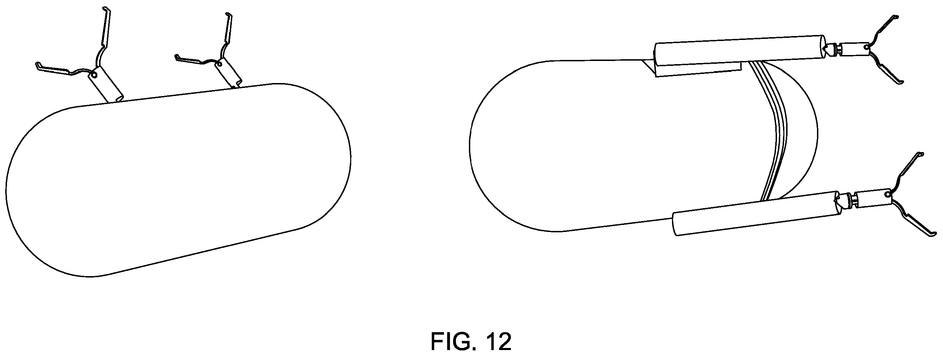

In some examples, the package (or pill case) may include structures for attaching the PBM pill to the inside of the GI lumen wall. One possible structure involves integrating one or more loops into the outside of the device (shown in FIG. 11; these loops can be used to suture the device to the mucosal wall with dissolvable sutures. Another possible structure involves clips which are integrated into the pill (e.g., the outside of the pill case); these clips are configured to grab a portion of the mucosal wall and lock closed, attaching the pill to the wall. A device may include one or more of these clips, positioned either at the ends or along the lateral axis of the pill (FIG. 12).

The system 100 also includes one or more devices that are outside the body (e.g., receiver 104 and user interface 106, collectively referred to as external device), which can be configured to record and display signals transmitted from the intraluminal device and to transmit control signals to the PBM pill 102. In some examples, the external device may also transmit power to the PBM pill 102 (for example, to charge the battery or to provide power to the PBM pill without charging the battery). For example, the external device can be a tablet with a graphical user interface that allows real-time control of PBM parameters including the power, wavelength, and timing of light therapy, and visualization of reflectance measurements. The external device may also allow visualization of data measured by sensors/transducers, including pH, temperature, pressure, impedance, or accelerometer measurements, the heartbeat, respiratory rate, or tissue oxygenation level, or the battery charge remaining. As another example, the external device may include a rendezvous device placed on the skin of the patient or worn on the patient's body (the receiver 104). The external device may relay data to a cloud-based server for remote visualization and control. The external device may also be used for calibration of the PBM therapy: reflectance signals off the walls can be used to adjust the light stimulus until it reaches a predetermined set point. This calibration procedure can involve adjusting the intensity of the light up or down until the amount of reflected light reaches a predetermined set point (e.g., determined for the specific person, for the specific disease condition, based on a standard for all users, etc.) entered using the graphical user interface; this can allow for fine-tuning of therapy in cases of differing albedo on the interior lumen. Another type of calibration can involve adjusting the wavelength of light emitted by the LED array until the reflectance value reaches a maximum, minimum, or a predetermined value set by a physician or user. For either of these calibration methods, the system can periodically test and adjust the PBM parameters to ensure that the stimulus stays within a predetermined threshold.

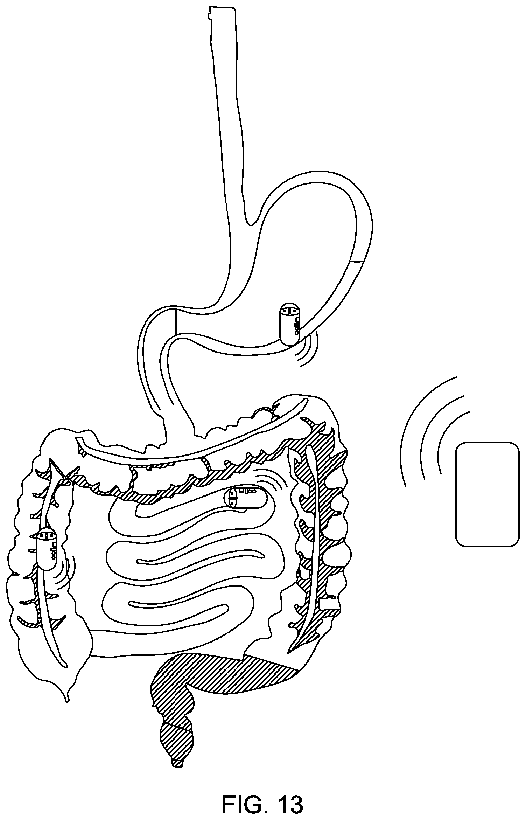

As shown in FIG. 13, multiple PBM pills 102 may be used to apply PBM therapy to multiple parts or the same part of the GI tract at once or in sequence. Wireless communication between the external device and/or between the PBM pills 102 may be used to coordinate the timing of therapy.

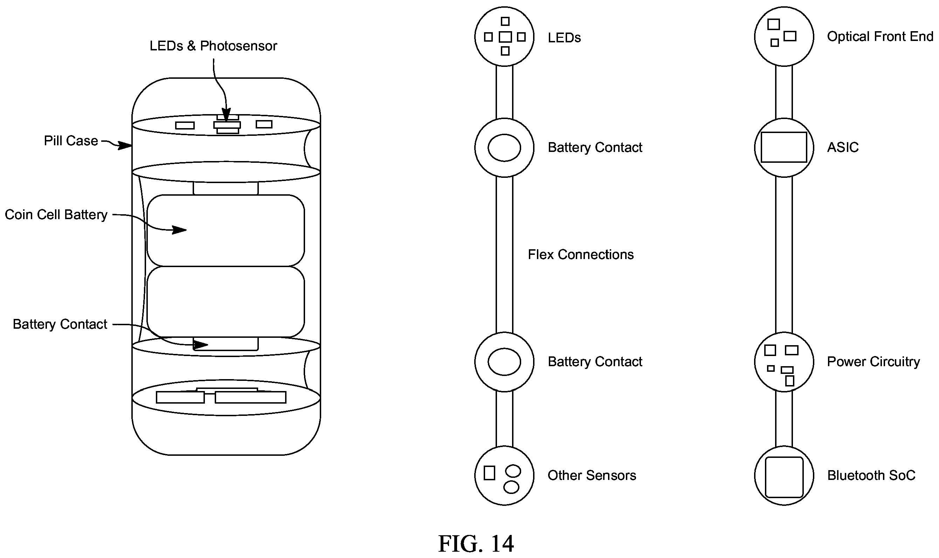

The PBM pill 102 can be manufactured with an electronic circuit that can be constructed of a rigid-flex printed circuit board (shown in FIG. 14), where components of the rigid-flex printed circuit board can be placed on a plurality of circular rigid sections of approximately 1 cm (this is just an example, which may be bigger or smaller depending on the size of the PBM pill) in diameter connected by flexible polyimide cables. The outer pill case 208 may be constructed in two parts by injection molding; for example, one part may be opaque and comprise the majority of the case (including suture loops), while the second part may be transparent (to allow for the light delivery and reflectance detection). To assemble the device, components may be soldered on to the rigid-flex PCB, then the PCB can be folded and inserted into the first section of the case. Battery cells can be placed in between two rigid sections such that the negative and positive terminals contact connectors on two different boards. Once in place, the second section of the case can be snapped or screwed into place to isolate the circuit. A gasket or o-ring can be used to prevent water intrusion between the two sections of the case.

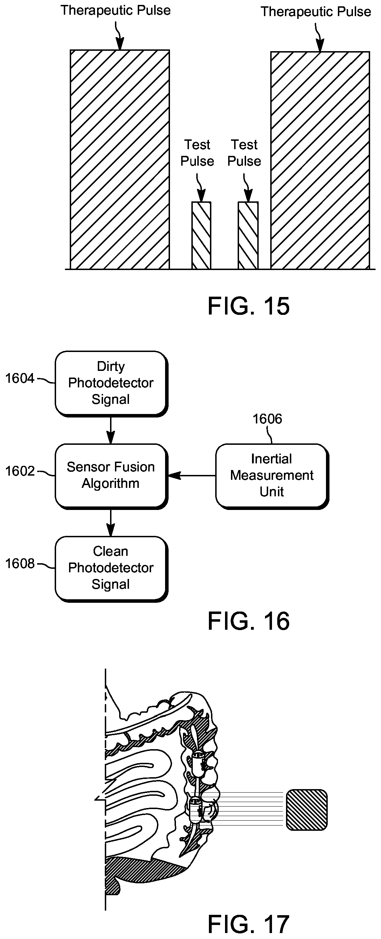

Example uses of the system 100 may include monitoring for hemorrhage within the GI tract or for tissue oxygen perfusion of the GI mucosa, either independently or while providing PBM therapy. To monitor tissue oxygen perfusion, reflectance from the tissue at two wavelengths, for example, one longer than 850nm and one shorter than 850nm for example can be measured and this signal can be used as part of a closed-loop therapy using PBM to increase tissue perfusion in the gastrointestinal lumen; pulses at a lower amplitude can be interspersed with therapeutic pulses to allow periodic measurement during therapy (for example, FIG. 15 shows low amplitude test pulses at two wavelengths between each PBM pulse). The signal processing electronics can then take the ratio of these two wavelengths during the systolic and diastolic portions of the cardiac cycle and apply an algorithm to determine blood oxygenation within the tissue proximal to the device. Tissue oxygenation can be used to monitor for anastomotic leakage, to diagnose shock, or to better understand the efficacy of the applied PBM therapy. To monitor for hemorrhage within the GI tract, the system 100 can measure the reflectance at multiple wavelengths and apply algorithms within the signal processing electronics to determine the amount of blood within the GI tract. As an example, a digital filter can be used to eliminate components of the reflectance signal associated with heartbeat, respiration, and movement, then the reflectance ratios between multiple wavelengths can be analyzed to determine the presence of hemoglobin inside the GI tract. One potential filtering method which may be implemented involves taking the fast Fourier transform of the signal and eliminating the dominant wavelength component in the expected range for heartbeat (0.8 Hz to 3.2 Hz) and respiration (0.15 Hz to 1 Hz), then reconstructing the signal in the time domain to perform further processing steps. In another example, an inertial measurement unit can be included in the PBM pill 102 circuitry to provide a secondary signal for heartbeat, breathing, and/or movement; sensor fusion algorithms can then be used to combine the inertial measurement unit signal with the photodiode signal to eliminate noise.

As shown in FIG. 16, for example, one or more sensor fusion algorithms 1602 can be used to combine a dirty photoreceptor signal 1604 (e.g., a noisy signal due to extraneous data, such as movement, vibration etc.) with a signal from an inertial measurement unit 1606, resulting in a clean (or at least cleaner) photodetector signal 1608. Wavelengths with differing penetration depth into the lumen can be used to remove signals dirtying the photoreceptor signal. As an example, feces within the GI mucosa can be such a dirtying factor, preventing the measurement of blood concentration or reflection from the GI lumen wall. However, the blood and feces have different reflectance at or around 850nm, which can be used to identify and disregard reflectance from fecal material within the lumen. The optical measurement of blood within the GI tract can be used to identify bleeding ulcers in order to time PBM treatment, to monitor for hemorrhage after abdominal surgery, or to track wound healing inside the GI lumen.

As shown in FIG. 17, the PBM pill 102 can be locally activated once it reaches a certain location/region in the GI tract to activate the PBM pill 102 when it reaches a certain location in the GI tract. For example, a device 1702 may be placed on a patient's skin above the therapy site. The patient can swallow the PBM pill 102, which can be configured to measure the magnitude of ambient light. The device 1702 can emit light (e.g., infrared light, either constant or pulsed with a known pattern to distinguish from other light sources) in a focused manner into the patient's GI tract. A rise in ambient light detected due to the infrared light emitted by the device 1702 would indicate that the PBM pill 102 has reached the target treatment location, at which point, the PBM pill 102 can begin providing PBM therapy. In one example, the focused light from the external optical device 1702 can be pulsed in such a way that a digital signal was transmitted to the PBM pill 102. In its simplest form this digital signal can be a pulse train at a known frequency. By filtering the ambient light to detect this frequency, interference from other environmental light sources can be mitigated and the PBM pill 102 can be more reliably activated in the desired region of the GI tract.

Several methods using the system 100 to treat various conditions are now described. It will be understood that these methods are just examples and are not meant to be exclusive. Additionally, the steps of the methods which are illustrated can be executed in different orders and/or with one or more of the steps not needed or additional steps added.

For any of these several methods, an intraluminal device also referred to as PBM pill or GI pill) may use reflectance measurements as well as measurements from transducers included in the device to monitor passage through the GI tract. For instance, reflectance differences between the lumen walls in the esophagus, stomach, small intestine, and colon can be used to identify rough location within the GI tract. Additional sensing modalities such as pH measurement, temperature measurement, impedance measurement, osmolarity measurement, or pressure measurement, can also be used to provide location along the GI tract. These additional measurements may be used to provide additional information to algorithms for detecting the presence of inflammation, the presence of ulcerated tissue, or the presence of bacteria. They may also be employed to verify that the device is operating correctly. To conserve power, the intraluminal device can wait to provide PBM therapy until the intraluminal device enters one portion of the GI tract. Location tracking also allows the intraluminal device to transmit the location of any detected inflammation, ulceration, or bacterial overgrowth event to an external interface outside the body; this information can be used by a physician to monitor the overall progression of a patient's condition.

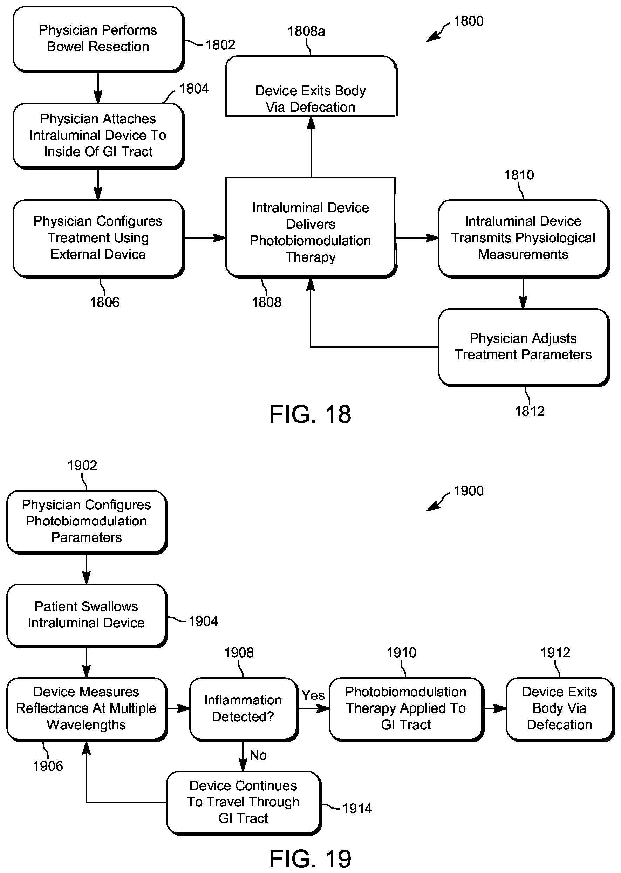

Referring now to FIG. 18, illustrated is a method 1800 for accelerating wound healing after bowel resection surgery (e.g., reduce inflammation, promote tissue oxygenation, and the like). The method is minimally invasive. At 1802, bowel resection surgery is performed (e.g., by a physician, a robot, or a combination of the physician and the robot). At 1804, an intraluminal device is attached to the inside of the GI tract. The device may, for example, be sutured in place using dissolvable sutures. At 1806, the treatment is configured using the external device (e.g., by setting PBM parameters including, but not limited to light intensity, wavelength, frequency, time of stimulation, etc.). The device may them be activated (e.g., by the physician, a therapist, the user, etc.). At 1808, the intraluminal device (or PBM pill) delivers PBM therapy (applying light according to the configuration) to the surgical wound from inside the GI tract. A photodiode within the PBM pill may also use reflected light from the PBM to monitor various aspects of wound healing, including but not limited to tissue oxygen perfusion, systemic oxygen perfusion, tissue inflammation, heart rate, and/or respiratory rate. After the delivery of the PBM therapy, either the device exits the body via defecation at 1808a or at 1810 the intraluminal device transmits physiological measurements to the external device. If the intraluminal device transmits physiological measurements to the external device, then, at 1812, the treatment can be adjusted based on the physiological measurements (adjustments can be made manually by a physician, a therapist, or a user or adjustments can be done automatically in a closed loop fashion). It should be noted that in any situation, the PBM device will eventually exit the body via defecation at 1810.

Referring now to FIG. 19, illustrated is a method 1900 that provides therapy for inflammatory bowel disease (IBD) using PBM. At 1902, the intraluminal device is activated, for example, by the user or physician, and programmed with appropriate PBM parameters. Then, at 1904, the patient swallows the intraluminal device. At 1906, once the intraluminal device has entered the GI tract, the intraluminal device measures reflectance at multiple wavelengths. For example, the intraluminal device will emit light at a lower intensity level, record the reflected signal from a section of the GI tract wall, and use signal processing algorithms to determine if inflammation is present on the section of the GI tract the wall. If inflammation is detected, then, at 1910, the intraluminal device will emit a higher level of light to provide PBM therapy to reduce inflammation of the section of the GI tract wall. In some examples, the intraluminal device may automatically apply PBM in response to detecting inflammation for closed-loop therapy. In other examples, the physician or user may be alerted to the presence of inflammation through communication from the intraluminal device to the external device and may choose to initiate PBM at that time. The PBM can be configured for stimulating the enteric nervous system or to reduce levels of TNF-alpha. The PBM can also be configured to increase nitric oxide production in the mucosa. Once the intraluminal device passes through the GI tract it will naturally exit the body during defecation at 1912. Additionally, at 1908, if inflammation is not detected, at 1914, the device continues to travel through the GI tract to a new location.

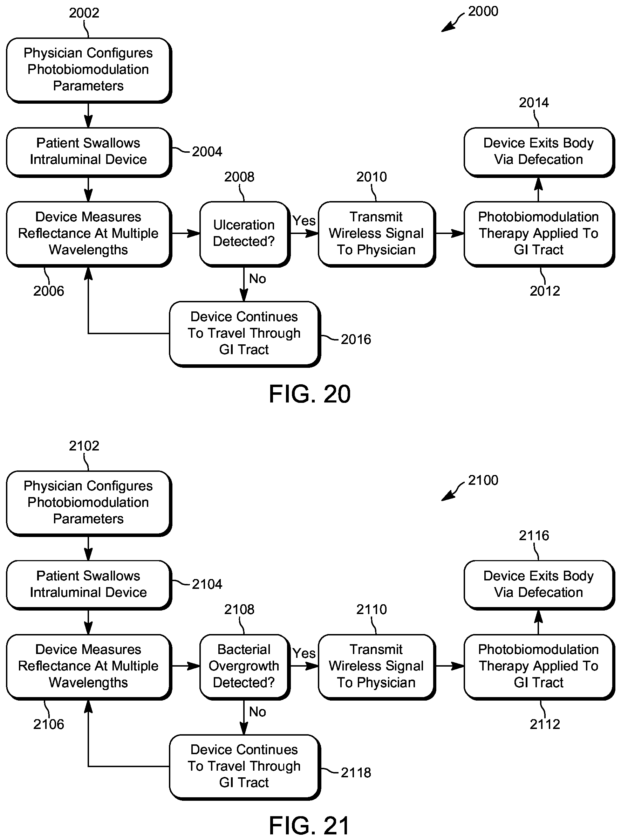

Referring now to FIG. 20, illustrated is a method 2000 for providing therapy for gastrointestinal ulcers using PBM. At 2002, therapeutic parameters of the intraluminal device are configured, for example, by a physician, a therapist, or the user. At 2004, the patient swallows the intraluminal device. Once the intraluminal device has entered the GI tract, at 2006, the intraluminal device will emit light at a lower intensity level, record the reflected signal from a section of the GI tract wall (this can be repeated at multiple wavelengths), and, at 2008, the intraluminal device can use signal processing algorithms to determine if there is ulceration in the mucosa. If an ulcer is detected, at 2010, the intraluminal device will emit a higher level of light to provide photobiomodulation therapy to reduce inflammation and accelerate healing. In some embodiments of the method the intraluminal device may automatically apply PBM in response to the ulcer for closed-loop therapy; in other embodiments, the physician or user may be alerted to the presence and location of ulceration through communication from the intraluminal device to the external device (at 2010) and may choose to initiate PBM at that time (at 2012). Once the intraluminal device passes through the GI tract it will naturally exit the body during defecation, at 2012. It should be noted that if ulceration is not detected at 2008, then the intraluminal device continues to travel through the GI tract at 2014 and is eventually defecated.

Referring now to FIG. 21, illustrated is a method 2100 of detecting and providing therapy for bacterial overgrowth. At 2102, the intraluminal device is activated, for example by the user or physician, and programmed with appropriate photobiomodulation parameters. Then at 2104 the patient swallows the intraluminal device. Once the intraluminal device has entered the GI tract, the intraluminal device will emit light at a lower intensity level, record the reflected signal from a section of the GI tract wall (at 2016), and use signal processing algorithms to quantify the bacterial load at each section of the GI tract wall (at 2110). In some examples, the intraluminal device can be coated in part or in whole with a coating that fluoresces in the presence of bacteria. If the concentration of bacteria reaches a certain threshold at a site, then the intraluminal device will emit a higher level of light, via fluorescence, to provide PBM therapy to reduce the bacteria at the site. In some examples, the intraluminal device can automatically apply PBM in response to bacterial concentration for closed-loop therapy; in other embodiments, the physician or user may be alerted to the presence of bacterial overgrowth through communication from the intraluminal device to the external device (at 2112) and may choose to initiate PBM at that time (at 2114). Once the intraluminal device passes through the GI tract it will naturally exit the body during defecation at 2116. It should be noted that if ulceration is not detected at 2108, the device continues to travel through the GI tract and eventually exit the body during defecation at 2116.

Referring now to FIG. 19, illustrated is a method 1900 that provides therapy for inflammatory bowel disease (IBD) using PBM. At 1902, the intraluminal device is activated by the user or physician and programmed with appropriate PBM parameters. Then, at 1904, the patient swallows the intraluminal device. At 1906, the intraluminal device measures reflectance at multiple wavelengths. For example, the intraluminal device can emit light at a lower level, record the reflected signal from the gastrointestinal tract wall, and use signal processing algorithms to determine if inflammation is present on the wall. If inflammation is detected, at 1908, at 1910, the intraluminal device can emit a higher level of light to provide PBM therapy to reduce inflammation of the site. In some examples, the intraluminal device may automatically apply PBM in response to detected inflammation for closed-loop therapy; in other examples, the physician or user may be alerted to the presence of inflammation through communication to the external device and may choose to initiate PBM at that time. The PBM may be configured for stimulating the enteric nervous system or to reduce levels of TNF-alpha. The PBM may also be configured to increase nitric oxide production in the mucosa. Once the intraluminal device passes through the GI tract it can naturally exit the body during defecation at 1912. Additionally, at 1908, if inflammation is not detected, at 1914, the device continues to travel through the GI tract.

Referring now to FIG. 20, illustrated is a method 2000 for providing therapy for gastrointestinal ulcers using PBM. At 2002, therapeutic parameters can be configured by a physician, a therapist, or the user. At 2004, the patient can swallow the intraluminal device. The intraluminal device can emit light at a lower level, record the reflected signal from the gastrointestinal tract wall (this can be repeated at multiple wavelengths at 2006), and use signal processing algorithms to determine if there is ulceration in the mucosa at 2008. If an ulcer is detected, at 2010, the intraluminal device can emit a higher level of light to provide photobiomodulation therapy to reduce inflammation and accelerate healing. In some examples the intraluminal device may automatically apply PBM in response to the ulcer for closed-loop therapy; in other examples, the physician or user may be alerted to the presence and location of ulceration through communication to the external device (at 2010) and may choose to initiate PBM at that time (at 2012). Once the intraluminal device passes through the GI tract it can naturally exit the body during defecation (at 2012). It should be noted that if ulceration is not detected at 2008, the device continues to travel through the GI tract at 2014.

Referring now to FIG. 21, illustrated is a method 2100 of detecting and providing therapy for bacterial overgrowth. At 2102, the intraluminal device is activated by the user or physician and programmed with appropriate photobiomodulation parameters. Then at 2104 the patient swallows the intraluminal device. The intraluminal device can emit light at a lower level, record the reflected signal from the gastrointestinal tract wall (at 2016), and use signal processing algorithms to quantify the bacterial load at each portion of the intestinal wall (at 2110). In some examples, the intraluminal device may be coated in part or in whole with a coating that fluoresces in the presence of bacteria. If the concentration of bacteria reaches a certain threshold, the intraluminal device can emit a higher level of light to provide PBM therapy to reduce the bacteria at the site. In some examples, the intraluminal device may automatically apply PBM in response to bacterial concentration for closed-loop therapy; in other examples, the physician or user may be alerted to the presence of bacterial overgrowth through communication to the external device (at 2112) and may choose to initiate PBM at that time (at 2114). Once the intraluminal device passes through the GI tract it can naturally exit the body during defecation at 2016. It should be noted that if ulceration is not detected at 2108, the device continues to travel through the GI tract at 2116.

References to "one example", "an example", "some examples" and so on, indicate that the embodiment(s) or example(s) so described may include a particular feature, structure, characteristic, property, element, or limitation, but that not every embodiment or example necessarily includes that particular feature, structure, characteristic, property, element, or limitation. Furthermore, repeated use of the phrase "in an aspect" and similar phrases does not necessarily refer to the same embodiment, though it may.

Where the disclosure or claims recite "a," "an," "a first," or "another" element, or the equivalent thereof, it should be interpreted to include one or more than one such element, neither requiring nor excluding two or more such elements. Furthermore, what have been described above are examples. It is, of course, not possible to describe every conceivable combination of components or methods, but one of ordinary skill in the art will recognize that many further combinations and permutations are possible.

## Claims

1. A system (100) comprising:
an intraluminal device (102) comprising:
at least one light delivery device (202) configured to provide photobiomodulation;
at least one photodetector device (204) configured to measure reflectance based on the photobiomodulation;
one or more sensors (206);
a processor (306) configured to receive a reflectance signal from the at least one photodetector device (204) based on the reflectance measured by the at least one photodetector device (204) and to process the reflectance signal, wherein the processor (306) is configured to receive signals from the one or more sensors (206) that are used by one or more sensor fusion algorithms to clean the reflectance signal to create the processed signal; and
a wireless transceiver (308) coupled to the processor (306) to transmit at least a portion of the processed reflectance signal; and
an external device (104) comprising a wireless transceiver (310) configured to receive at least the portion of the processed reflectance signal.

2. The system of claim 1, wherein the intraluminal device is configured to travel through at least a portion of a patient's gastrointestinal tract and the external device is configured to be outside the patient's body.

3. The system of claim 1, wherein at least the processor and the wireless transceiver are encased in a biocompatible housing (208).

4. The system of claim 1, wherein the intraluminal device further comprises one or more sensors configured to provide data regarding an environment of the intraluminal device.

5. The system of claim 1, wherein the wireless transceiver of the external device is configured to send configuration information and/or parameters for the photobiomodulation to the wireless transceiver of the processor and/or to the at least one light delivery device.

6. The system of claim 1, wherein the processor is configured to control delivery of the photobiomodulation provided by the at least one light delivery device based on the processed reflectance signal.

7. The system of claim 1, wherein the external device is configured to display a graphical, tactile, or audio representation of the at least the portion of the processed reflectance signal.

8. The system of claim 1, wherein the at least one light delivery device comprises at least four light delivery devices, wherein at least two of the at least four light delivery devices are configured to provide light having different wavelengths from one another.

9. The system of claim 1, wherein the at least one light delivery device comprises a light emitting diode or a laser diode.

10. The system of claim 1, wherein the at least one photodetector device comprises at least one of a photosensor, a photodiode, a photoresistor, and a phototransistor.

11. The system of claim 1, wherein the intraluminal device comprises a biocompatible casing (208) configured to encase at least a portion of:
the at least one light delivery device configured to provide photobiomodulation;
the at least one photodetector device configured to measure reflectance based on the photobiomodulation;
the one or more sensors;
the processor configured to receive a reflectance signal from the at least one photodetector device based on the reflectance measured by the at least one photodetector device and to process the reflectance signal, wherein the processor is configured to receive signals from the one or more sensors that are used by one or more sensor fusion algorithms to clean the reflectance signal to create the processed signal; and
the wireless transceiver coupled to the processor to transmit at least a portion of the processed reflectance signal.

12. The intraluminal device of claim 12, wherein at least a portion of the biocompatible casing is opaque.

13. The system claim 12, wherein a portion of the at least one light delivery device and/or the at least one photodetector device are arranged on an outer surface of the biocompatible casing.

14. The system of claim 12, wherein the at least one light delivery device and/or the at least one photodetector device is encased within a transparent portion of the biocompatible casing.

## Patentansprüche

1. System (100), das Folgendes umfasst:
eine intraluminale Vorrichtung (102), umfassend:
mindestens eine Lichtabgabevorrichtung (202), die dazu konfiguriert ist, Fotobiomodulation bereitzustellen;
mindestens eine Fotodetektorvorrichtung (204), die dazu konfiguriert ist, Reflexion basierend auf der Fotobiomodulation zu messen;
einen oder mehrere Sensoren (206);
einen Prozessor (306), der dazu konfiguriert ist, ein Reflexionssignal von der mindestens einen Fotodetektorvorrichtung (204) basierend auf der von der mindestens einen Fotodetektorvorrichtung (204) gemessenen Reflexion zu empfangen und das Reflexionssignal zu verarbeiten, wobei der Prozessor (306) dazu konfiguriert ist, Signale von dem einen oder den mehreren Sensoren (206) zu empfangen, die von einem oder mehreren Sensorfusionsalgorithmen verwendet werden, um das Reflexionssignal zu bereinigen, um das verarbeitete Signal zu erzeugen; und
einen drahtlosen Sendeempfänger (308), der mit dem Prozessor (306) gekoppelt ist, um mindestens einen Teil des verarbeiteten Reflexionssignals zu übertragen; und
eine externe Vorrichtung (104), die einen drahtlosen Sendeempfänger (310) umfasst, der dazu konfiguriert ist, mindestens den Teil des verarbeiteten Reflexionssignals zu empfangen.

2. System nach Anspruch 1, wobei die intraluminale Vorrichtung dazu konfiguriert ist, durch mindestens einen Teil eines Magen-Darm-Trakts eines Patienten zu gehen, und die externe Vorrichtung dazu konfiguriert ist, sich außerhalb des Körpers des Patienten zu befinden.

3. System nach Anspruch 1, wobei mindestens der Prozessor und der drahtlose Sendeempfänger in einem biokompatiblen Gehäuse (208) untergebracht sind.

4. System nach Anspruch 1, wobei die intraluminale Vorrichtung weiter einen oder mehrere Sensoren umfasst, die dazu konfiguriert sind, Daten hinsichtlich einer Umgebung der intraluminalen Vorrichtung bereitzustellen.

5. System nach Anspruch 1, wobei der drahtlose Sendeempfänger der externen Vorrichtung dazu konfiguriert ist, Konfigurationsinformationen und/oder Parameter für die Fotobiomodulation an den drahtlosen Sendeempfänger des Prozessors und/oder an die mindestens eine Lichtabgabevorrichtung zu senden.

6. System nach Anspruch 1, wobei der Prozessor dazu konfiguriert ist, Abgabe der von der mindestens einen Lichtabgabevorrichtung bereitgestellten Fotobiomodulation basierend auf dem verarbeiteten Reflexionssignal zu steuern.

7. System nach Anspruch 1, wobei die externe Vorrichtung dazu konfiguriert ist, eine grafische, taktile oder akustische Darstellung des mindestens des Teils des verarbeiteten Reflexionssignals anzuzeigen.

8. System nach Anspruch 1, wobei die mindestens eine Lichtabgabevorrichtung mindestens vier Lichtabgabevorrichtungen umfasst, wobei mindestens zwei der mindestens vier Lichtabgabevorrichtungen dazu konfiguriert sind, Licht mit voneinander unterschiedlichen Wellenlängen bereitzustellen.

9. System nach Anspruch 1, wobei die mindestens eine Lichtabgabevorrichtung eine Leuchtdiode oder eine Laserdiode umfasst.

10. System nach Anspruch 1, wobei die mindestens eine Fotodetektorvorrichtung mindestens einen Fotosensor, eine Fotodiode, einen Fotowiderstand und einen Fototransistor umfasst.

11. System nach Anspruch 1, wobei die intraluminale Vorrichtung ein biokompatibles Gehäuse (208) umfasst, das dazu konfiguriert ist, mindestens einen Teil von Folgendem unterzubringen:
die mindestens eine Lichtabgabevorrichtung, die dazu konfiguriert ist, eine Fotobiomodulation bereitzustellen;
die mindestens eine Fotodetektorvorrichtung, die dazu konfiguriert ist, Reflexion basierend auf der Fotobiomodulation zu messen;
den einen oder die mehreren Sensoren;
den Prozessor, der dazu konfiguriert ist, ein Reflexionssignal von der mindestens einen Fotodetektorvorrichtung basierend auf der von der mindestens einen Fotodetektorvorrichtung gemessenen Reflexion zu empfangen und das Reflexionssignal zu verarbeiten, wobei der Prozessor dazu konfiguriert ist, Signale von dem einen oder den mehreren Sensoren zu empfangen, die von einem oder mehreren Sensorfusionsalgorithmen verwendet werden, um das Reflexionssignal zu bereinigen, um das verarbeitete Signal zu erzeugen; und
der drahtlose Sendeempfänger mit dem Prozessor gekoppelt ist, um mindestens einen Teil des verarbeiteten Reflexionssignals zu übertragen.

12. Intraluminale Vorrichtung nach Anspruch 12, wobei mindestens ein Teil des biokompatiblen Gehäuses undurchsichtig ist.

13. System nach Anspruch 12, wobei ein Teil der mindestens einen Lichtabgabevorrichtung und/oder der mindestens einen Fotodetektorvorrichtung auf einer Außenfläche des biokompatiblen Gehäuses angeordnet sind.

14. System nach Anspruch 12, wobei die mindestens eine Lichtabgabevorrichtung und/oder die mindestens eine Fotodetektorvorrichtung in einem transparenten Teil des biokompatiblen Gehäuses untergebracht sind.

## Revendications

1. Système (100) comprenant :
Dispositif intraluminal (102), comprenant :
au moins un dispositif d'administration de lumière (202) configuré pour fournir une photobiomodulation ;
au moins un dispositif photodétecteur (204) configuré pour mesurer la réflectance sur la base de la photobiomodulation ;
un ou plusieurs capteurs (206) ;
un processeur (306) configuré pour recevoir un signal de réflectance provenant du au moins un dispositif photodétecteur (204) sur la base de la réflectance mesurée par le au moins un dispositif photodétecteur (204) et pour traiter le signal de réflectance, dans lequel le processeur (306) est configuré pour recevoir des signaux provenant des un ou plusieurs capteurs (206) qui sont utilisés par un ou plusieurs algorithmes de fusion de capteurs afin de nettoyer le signal de réflectance de manière à créer le signal traité ; et
un émetteur-récepteur sans fil (308) couplé au processeur (306) pour transmettre au moins une partie du signal de réflectance traité ; et
un dispositif externe (104) comprenant un émetteur-récepteur sans fil (310) configuré pour recevoir au moins la partie du signal de réflectance traité.

2. Système selon la revendication 1, dans lequel le dispositif intraluminal est configuré pour traverser au moins une partie du tractus gastro-intestinal d'un patient et le dispositif externe est configuré pour être à l'extérieur du corps du patient.

3. Système selon la revendication 1, dans lequel au moins le processeur et l'émetteur-récepteur sans fil sont enfermés dans un boîtier biocompatible (208).

4. Système selon la revendication 1, dans lequel le dispositif intraluminal comprend en outre un ou plusieurs capteurs configurés pour fournir des données concernant un environnement du dispositif intraluminal.

5. Système selon la revendication 1, dans lequel l'émetteur-récepteur sans fil du dispositif externe est configuré pour envoyer des informations de configuration et/ou des paramètres destinés à la photobiomodulation vers l'émetteur-récepteur sans fil du processeur et/ou vers le au moins un dispositif d'administration de lumière.

6. Système selon la revendication 1, dans lequel le processeur est configuré pour commander l'administration de la photobiomodulation fournie par le au moins un dispositif d'administration de lumière sur la base du signal de réflectance traité.

7. Système selon la revendication 1, dans lequel le dispositif externe est configuré pour afficher une représentation graphique, tactile ou audio d'au moins la partie du signal de réflectance traité.

8. Système selon la revendication 1, dans lequel le au moins un dispositif d'administration de lumière comprend au moins quatre dispositifs d'administration de lumière, dans lequel au moins deux des au moins quatre dispositifs d'administration de lumière sont configurés pour fournir une lumière présentant des longueurs d'onde différentes les unes des autres.

9. Système selon la revendication 1, dans lequel le au moins un dispositif d'administration de lumière comprend une diode électroluminescente ou une diode laser.

10. Système selon la revendication 1, dans lequel le au moins un dispositif photodétecteur comprend au moins un élément parmi un photocapteur, une photodiode, une photorésistance et un phototransistor.

11. Système selon la revendication 1, dans lequel le dispositif intraluminal comprend un boîtier biocompatible (208) configuré pour enfermer au moins une partie du :
au moins un dispositif d'administration de lumière configuré pour fournir une photobiomodulation ;
au moins un dispositif photodétecteur configuré pour mesurer la réflectance sur la base de la photobiomodulation ;
un ou plusieurs capteurs ;
le processeur étant configuré pour recevoir un signal de réflectance provenant du au moins un dispositif photodétecteur sur la base de la réflectance mesurée par le au moins un dispositif photodétecteur et pour traiter le signal de réflectance, dans lequel le processeur est configuré pour recevoir des signaux provenant des un ou plusieurs capteurs qui sont utilisés par un ou plusieurs algorithmes de fusion de capteurs afin de nettoyer le signal de réflectance de manière à créer le signal traité ; et
l'émetteur-récepteur sans fil couplé au processeur pour transmettre au moins une partie du signal de réflectance traité.

12. Dispositif intraluminal selon la revendication 12, dans lequel au moins une partie du boîtier biocompatible est opaque.

13. Système selon la revendication 12, dans lequel une partie du au moins un dispositif d'administration de lumière et/ou du au moins un dispositif photodétecteur sont agencés sur une surface extérieure du boîtier biocompatible.

14. Système selon la revendication 12, dans lequel le au moins un dispositif d'administration de lumière et/ou le au moins un dispositif photodétecteur sont enfermés dans une partie transparente du boîtier biocompatible.
